# EUROPÄISCHE PATENTSCHRIFT

(11) **EP 0 767 785 B1**
(45) Veröffentlichungstag und Bekanntmachung des Hinweises auf die Patenterteilung: **28.10.1998**
(21) Anmeldenummer: 95924255.3
(22) Anmeldetag: 16.06.1995
(51) Int. Cl.: C07D 301/00

(54) **VERFAHREN ZUR HERSTELLUNG VON 1,2-BUTYLENOXID**
METHOD OF PRODUCING 1,2-BUTYLENE OXIDE
PROCEDE DE PREPARATION DE 1,2-OXYDE DE BUTYLENE

(30) Priorität: 27.06.1994 DE 4422046
(43) Veröffentlichungstag der Anmeldung: 16.04.1997
(73) Patentinhaber: BASF AKTIENGESELLSCHAFT, 67056 Ludwigshafen (DE)
(72) Erfinder: LINGELBACH, Peter, D-67112 Mutterstadt (DE); ROSER, Joachim, D-68165 Mannheim (DE); SIGWART, Christoph, D-69198 Schriesheim (DE); SCHNURR, Werner, D-67273 Herxheim (DE); WEYER, Hans-Jürgen, D-67240 Bobenheim-Roxheim (DE)
(86) Internationale Anmeldenummer: EP9502337
(87) Internationale Veröffentlichungsnummer: WO9600222

(56) Entgegenhaltungen:
- US-A- 5 077 418
- US-A- 5 117 013
- J. AM. CHEM. SOC., Bd.83, 19. Oktober 0 Seiten 3096 - 3113 D. S. TARBELL ET AL. 'The Chemistry of Fumagillin'
- ZH. OBSHCH. KHIM., Seiten 3046 - 3051 M. A. AIZIKOVICH ET AL. 'Catalytic Hydrogenation of Vinylethylene Oxides. I. Hydrogenation of Butadiene Oxide'

## Beschreibung

Die vorliegende Erfindung betrifft ein Verfahren zur Herstellung von 1,2-Butylenoxid durch die katalytische Hydrierung von Vinyloxiran

Die katalytische Hydrierung von Vinyloxiran an Palladium auf Aluminiumoxid- und Palladium auf Aktivkohle-Katalysatoren ist aus US-A 5 077 418 und US-A 5 117 013 sowie aus Neftekhimiya 33, 131 (1993) bekannt. Die Ausbeuten, Selektivitäten und Umsätze in diesem Verfahren sind unbefriedigend.

Der vorliegenden Erfindung lag die Aufgabe zugrunde, ein Verfahren zur Herstellung von 1,2-Butylenoxid zu finden, das die Herstellung von 1,2-Butylenoxid aus Vinyloxiran mit besseren Umsätzen, Ausbeuten und Selektivitäten als im Stand der Technik ermöglicht.

Dementsprechend wurde ein Verfahren zur Herstellung von 1,2-Butylenoxid durch die katalytische Hydrierung von Vinyloxiran gefunden, das dadurch gekennzeichnet ist, daß man einen Palladium-Katalysator auf einem Träger aus Bariumsulfat, Zirkoniumdioxid oder Titandioxid oder einen Rhenium-haltigen Palladium-Trägerkatalysator verwendet.

Zur Durchführung des erfindungsgemäßen Verfahrens wird das Vinyloxiran oder Lösungen des Vinyloxirans in einem unter den Reaktionsbedingungen inerten Lösungsmittel in Gegenwart der erfindungsgemäß zu verwendenden Katalysatoren bei Temperaturen von im allgemeinen -10 bis 100°C, vorzugsweise -5 bis 50°C und besonders bevorzugt 0 bis 30°C bei einem Druck von 1 bis 100 bar, vorzugsweise 1 bis 50 bar, insbesondere 1 bis 30 bar hydriert.

Das erfindungsgemäße Verfahren kann ohne Lösungsmittel oder vorteilhaft in Anwesenheit eines unter den Reaktionsbedingungen inerten Lösungsmittels durchgeführt werden, beispielsweise in Gegenwart von Alkoholen, wie Methanol, Ethanol, Propanol, Isopropanol, n-Butanol, Isobutanol oder tert.-Butanol, Kohlenwasserstoffen, wie Petrolether, Benzol, Toluol oder Xylol, oder dipolar-aprotischen Lösungsmitteln, wie N-Alkyllactamen, z.B. N-Methylpyrrolidon oder N-Octylpyrrolidon, oder besonders bevorzugt Ethern, wie Tetrahydrofuran, Dioxan, Methyl-tert.-butylether, Di-n-butylether, Dimethoxyethan, Diethylether oder Diisopropylether.

Das erfindungsgemäße Verfahren kann sowohl kontinuierlich als auch diskontinuierlich, in der Gasphase oder in der flüssigen Phase ausgeübt werden. Bei der kontinuierlichen Betriebsweise können beispielsweise Rohrreaktoren oder Reaktorkaskaden eingesetzt werden, die in der Sumpf- oder Rieselfahrweise betrieben werden können. Vorteilhaft werden in diesen Reaktoren die Katalysatoren in Form eines Festbetts angeordnet. Bei der diskontinuierlichen Betriebsweise kann der Katalysator z.B. in Rührreaktoren in suspendierter Form oder vorteilhaft in einem Festbett angeordnet, z.B. bei Verwendung von Schlaufenreaktoren, verwendet werden.

Der Palladiumgehalt der erfindungsgemäß anwendbaren Katalysatoren beträgt im allgemeinen 0,1 bis 5 Gew.-%, vorzugsweise 0,3 bis 3 Gew.-% und besonders bevorzugt 0,5 bis 2 Gew.-%, berechnet als Pd und bezogen auf das Gesamtgewicht des Katalysators.

Die erfindungsgemäß einsetzbaren Rhenium-haltigen Palladium-Trägerkatalysatoren enthalten das Rhenium bezüglich des Palladiums in einem Gewichtsverhältnis von im allgemeinen 1 bis 99, vorzugsweise von 10 bis 90 und besonders bevorzugt von 40 bis 60.

Außer den für die allein Palladium als Aktivkomponente enthaltenden Katalysatoren vorteilhaft anwendbaren Trägermaterialien Bariumsulfat, Zirkoniumdioxid oder Titandioxid, können für die Rhenium-haltigen Palladium-Trägerkatalysatoren auch andere Trägermaterialien, wie Aktivkohle, Siliciumdioxid, Kieselgel oder Kieselgur vorteilhaft verwendet werden.

Die Herstellung der Palladiumkatalysatoren bzw. der Palladium- und Rhenium-haltigen Katalysatoren kann auf an sich herkömmliche Weise erfolgen, beispielsweise indem man die Trägermaterialien mit wäßrigen Lösungen wasserlöslicher Verbindungen des Palladiums bzw. des Palladiums und Rheniums imprägniert, beispielsweise mit den Nitraten, Sulfaten, Halogeniden, Carboxylaten des Palladiums bzw. Rheniums, den imprägnierten Träger z.B. bei Temperaturen von 50 bis 200°C, vorzugsweise 100 bis 150°C trocknet, anschließend bei Temperaturen von im allgemeinen 200 bis 600°C, vorzugsweise bei 300 bis 500°C, calciniert und den so erhaltenen Katalysator durch Behandlung mit Reduktionsmitteln, wie Hydrazin, Wasserstoff oder Wasserstoff-haltigen Gasen reduziert, beispielsweise bei Temperaturen von 100 bis 300°C, vorzugsweise bei 150 bis 250°C. Bei der Reduktion und Aktivierung des Katalysators mit Wasserstoff wird im allgemeinen so lange reduziert, bis keine wesentlichen Mengen an Wasser mehr gebildet werden.

Die Reduktion und Aktivierung der Katalysatoren mit Wasserstoff kann in situ im Hydrierreaktor während der Hydrierumsetzung erfolgen, vorzugsweise werden die Katalysatoren vor ihrem Einsatz im erfindungsgemäßen Verfahren reduziert und aktiviert.

Bei Verwendung thermisch instabiler Salze des Palladiums oder Rheniums zur Imprägnierung des Trägermaterials kann die Aktivierung des Katalysators auch thermisch durch Erhitzen des imprägnierten Trägers auf Temperaturen von im allgemeinen 100 bis 700°C, vorzugsweise von 200 bis 600°C, erfolgen.

Anstelle der obengenannten Palladium- bzw. Rheniumsalze können auch wasserlösliche Palladiumkomplexverbindungen oder wasserlösliche Salze der Sauerstoffsäuren des Rheniums, beispielsweise Alkalimetall- oder Ammonium-Rhenate oder -Perrhenate sowie Lösungen des Rheniumheptoxids (Re₂O₇) zur Imprägnierung des Trägers verwendet werden.

Bei der Herstellung Palladium- und Rhenium-haltiger Trägerkatalysatoren können zur Imprägnierung des Trägermaterials die Elemente Rhenium und Palladium gleichzeitig oder sukzessive auf das Trägermaterial aufgebracht werden.

Die Aufarbeitung der Reaktionsmischung zur Isolierung des 1,2-Butylenoxids kann auf herkömmliche Weise, z.B. durch Destillation erfolgen.

Das als Ausgangsmaterial benötigte Vinyloxiran kann z.B. nach dem Verfahren von US-A 4 897 498 durch partielle Oxidation von 1,3-Butadien an Silberkatalysatoren hergestellt werden.

1,2-Butylenoxid findet z.B. als Kraftstoffadditiv oder als Stabilisator von Chlorkohlenwasserstoffen Verwendung.

### Beispiele

Die Hydrierung des Vinyloxirans zu 1,2-Butylenoxid wurde in einem 50 ml-Rührautoklaven durchgeführt. Der Reaktor wurde mit jeweils 0,1 g des betreffenden Katalysators und 2,5 g Vinyloxiran in 22,5 g Tetrahydrofuran beschickt.

Die Hydrierbedingungen Temperatur, Druck, Verweilzeit und die Hydrierergebnisse (ermittelt mittels kalibrierter Gaschromatographie des Hydrieraustrags) sind in der Tabelle aufgelistet.

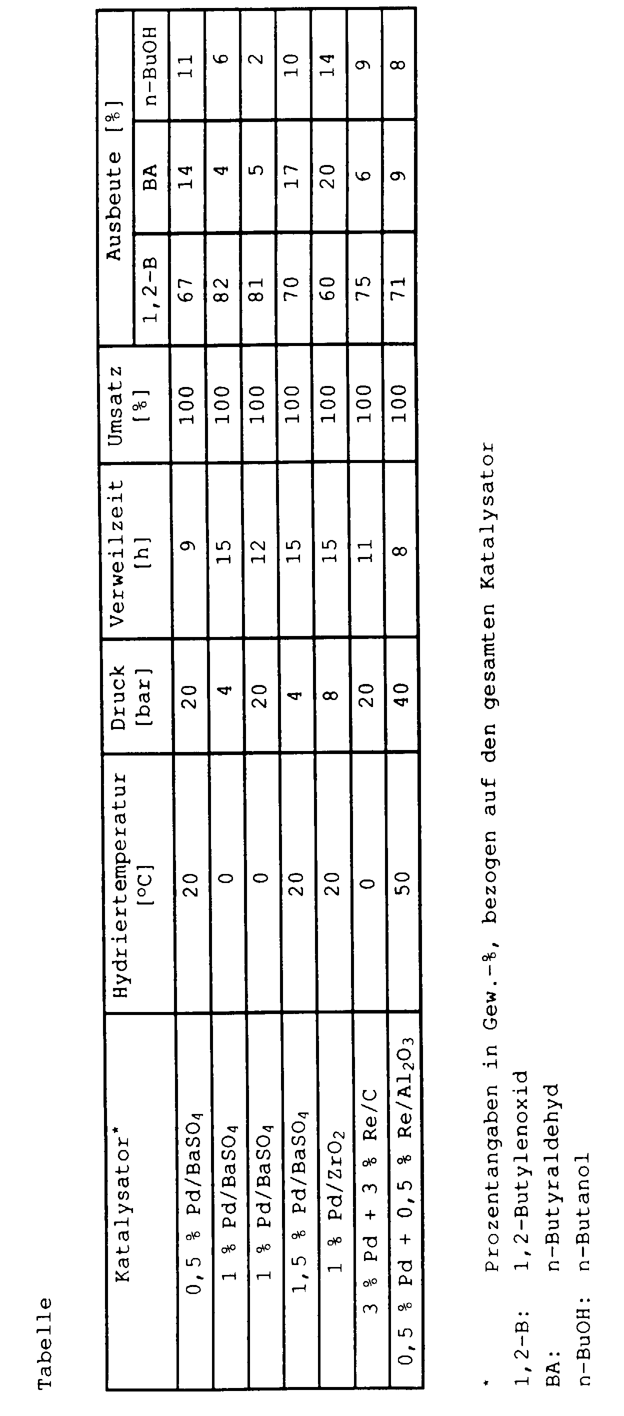

## Patentansprüche

1. Verfahren zur Herstellung von 1,2-Butylenoxid durch die katalytische Hydrierung von Vinyloxiran, dadurch gekennzeichnet, daß man einen Palladium-Katalysator auf einem Träger aus Bariumsulfat, Zirkoniumdioxid oder Titandioxid oder einen Rhenium-haltigen Palladium-Trägerkatalysator verwendet.

2. Verfahren nach Anspruch 1, dadurch gekennzeichnet, daß man einen Palladium-Katalysator mit einem Palladiumgehalt von 0,1 bis 5 Gew.-% verwendet.

3. Verfahren nach den Ansprüchen 1 und 2, dadurch gekennzeichnet, daß man einen Palladium-Katalysator mit einem Palladiumgehalt von 0,3 bis 3 Gew.-% verwendet.

4. Verfahren nach den Ansprüchen 1 bis 3, dadurch gekennzeichnet, daß man die Hydrierung bei einer Temperatur von -10 bis 100°C und bei einem Druck von 1 bis 100 bar durchführt.

5. Verfahren nach den Ansprüchen 1 bis 4, dadurch gekennzeichnet, daß man die Hydrierung in einem Lösungsmittel durchführt.

6. Verfahren nach den Ansprüchen 1 bis 5, dadurch gekennzeichnet, daß man die Hydrierung in einem dipolar, aprotischen Lösungsmittel durchführt.

## Claims

1. A process for preparing 1,2-butylene oxide by catalytic hydrogenation of vinyloxirane, which comprises using a palladium catalyst on a support comprising barium sulfate, zirconia or titania or a rhenium-containing supported palladium catalyst.

2. A process as claimed in claim 1, wherein the palladium catalyst contains from 0.1 to 5 % by weight of palladium.

3. A process as claimed in claim 1 or 2, wherein the palladium catalyst contains from 0.3 to 3 % by weight of palladium.

4. A process as claimed in any of claims 1 to 3, wherein the hydrogenation is carried out at from -10 to 100°C and at from 1 to 100 bar.

5. A process as claimed in any of claims 1 to 4, wherein the hydrogenation is carried out in a solvent.

6. A process as claimed in any of claims 1 to 5, wherein the hydrogenation is carried out in a dipolar aprotic solvent.

## Revendications

1. Procédé de préparation de 1,2-oxyde de butylène par hydrogénation catalytique de vinyloxiranne, caractérisé en ce que l'on utilise un catalyseur au palladium sur un support en sulfate de baryum, en dioxyde de zirconium ou en dioxyde de titane ou un catalyseur supporté au palladium contenant du rhénium.

2. Procédé selon la revendication 1, caractérisé en ce que l'on utilise un catalyseur au palladium avec une teneur en palladium de 0,1 à 5% en poids.

3. Procédé selon les revendications 1 et 2, caractérisé en ce que l'on utilise un catalyseur au palladium avec une teneur en palladium de 0,3 à 3% en poids.

4. Procédé selon l'une quelconque des revendications 1 à 3, caractérisé en ce que l'on mène l'hydrogénation à une température de -10 à 100°C et sous une pression de 1 à 100 bar.

5. Procédé selon l'une quelconque des revendications 1 à 4, caractérisé en ce que l'on mène l'hydrogénation dans un solvant.

6. Procédé selon l'une quelconque des revendications 1 à 5, caractérisé en ce que l'on mène l'hydrogénation dans un solvant aprotique dipolaire.
